# EUROPÄISCHE PATENTANMELDUNG

(11) **EP 3 590 493 A1**
(43) Veröffentlichungstag der Anmeldung: **08.01.2020**
(21) Anmeldenummer: 18181832.9
(22) Anmeldetag: 05.07.2018
(51) Int. Cl.: A61K 8/34, A61K 8/41, A61Q 5/12

(54) **HYBRID QUATS ZUR HAARPFLEGE**

(71) Anmelder: Evonik Operations GmbH, 45128 Essen (DE)
(72) Erfinder: SCHWAB, Peter, 45133 Essen (DE); NEUBAUER, Stefan, 50931 Köln (DE); WINTER, Patrick, 45473 Mülheim an der Ruhr (DE)
(74) Vertreter: Evonik Patent Association

(57) **Zusammenfassung**

Gegenstand der Erfindung sind Zusammensetzungen enthaltend
A) mindestens ein Hybridquat und
B) mindestens einen Fettalkohol.

## Beschreibung

### Gebiet der Erfindung

Gegenstand der Erfindung sind Zusammensetzungen enthaltend
A) mindestens ein Hybridquat und
B) mindestens einen Fettalkohol.

### Stand der Technik

Alkylquats und Esterquats werden in kosmetischen Formulierungen als Haarkonditioniermittel eingesetzt. Beide haben bestimmte Nachteile: Alkylquats, insbesondere Dialkylquats, sind generell schwer biologisch abbaubar und belasten die Umwelt. Esterquats, insbesondere TEA-Esterquats, sind hydrolyseempfindlich und können deshalb nur eingeschränkt in wässrigen Formulierungen eingesetzt werden.

Es konnte nun gezeigt werden, dass Alkylesterquats (Hybridquats) bestehend aus einem Ammoniumsalz mit mindestens einer Fettalkylkette und mindestens einer mit Fettsäure veresterten Estergruppe hervorragende Konditioniereigenschaften besitzen und gleichzeitig die Nachteile der Alkylquats beziehungsweise Esterquats nicht aufweisen.

Zusätzlich wurde überraschenderweise gefunden, dass die Alkylesterquats in der Lage sind, außergewöhnlich hohe Viskositäten in Formulierungen mit Fettalkohol aufzubauen.

Die DE3623215 offenbart haarkosmetische Zusammensetzungen enthaltend hochethoxylierte Hybridquats und Fettalkohol.

Die DE2928603 offenbart Hybridquats und deren Verwendung als Wäscheweichspüler.

Aufgabe der Erfindung war es, alternative Haarkonditioniermittel auf Basis von quaternisierten Ammoniumverbindungen bereitzustellen.

### Beschreibung der Erfindung

Überraschenderweise wurde gefunden, dass die im Folgenden beschriebenen Hybridquats die der Erfindung gestellte Aufgabe zu lösen vermögen.

Gegenstand der vorliegenden Erfindung sind daher Zusammensetzungen enthaltend bestimmte Hybridquats und Fettalkohol.

Ein weiterer Gegenstand der Erfindung ist die Verwendung von den bestimmten Hybridquats zur Konditionierung von Haut und keratinischen Fasern, insbesondere Haaren.

Ein weiterer Gegenstand der Erfindung ist die Verwendung von den Hybridquats zur Viskositätserhöhung von fettalkoholhaltigen Zusammensetzungen.

Noch ein Gegenstand der vorliegenden Erfindung ist die Verwendung von den Hybridquats als Emulgator, insbesondere kationischer Emulgator, in bevorzugt kosmetischen Formulierungen.

Ein Vorteil der vorliegenden Erfindung ist es, dass die erfindungsgemäße Zusammensetzung überwiegend auf Basis natürlicher Rohstoffe bereitgestellt werden kann.

Ein weiterer Vorteil ist, dass die eingesetzten Hybridquats über eine verbesserte Hydrolysestabilität verfügen.

Ein weiterer Vorteil ist, dass die eingesetzten Hybridquats über eine verbesserte thermische Stabilität verfügen.

Noch ein Vorteil der vorliegenden Erfindung ist es, dass eine gute Konditionierleistung ohne negativen Einfluss auf die Formulierungsviskosität erreicht werden kann.

Ein weiterer Vorteil ist, dass die eingesetzten Hybridquats sich hervorragend verarbeiten lassen. Sie sind insbesondere in einer Vielzahl unterschiedlicher Formulierungen einsetzbar.

Ein weiterer Vorteil ist, dass die eingesetzten Hybridquats die Viskositäten von auf Fettalkoholbasierten Suspensionen, wie sie üblicherweise in Conditioner Rinse Formulierungen eingesetzt werden, im Vergleich zu Alkylquats und Esterquats deutlich erhöhen. Somit kann der Fettalkoholgehalt reduziert werden, was die Gesamtkosten der Formulierung reduziert.

Ein weiterer Vorteil ist, dass die eingesetzten Hybridquats die auf Fettalkohol basierten Suspensionen auch ohne einen weiteren Emulgator ausreichend stabilisieren.

Ein weiterer Vorteil ist, dass die eingesetzten Hybridquats sich auch in Shampoos einsetzen lassen und dabei konditionierend aktiv sind.

Ein weiterer Vorteil ist, dass die eingesetzten Hybridquats gute Bioabbaubarkeit aufweisen.

Noch ein Vorteil der der vorliegenden Erfindung ist es, dass der Glanz der behandelten keratinischen Fasern gesteigert wird.

Ein weiterer Vorteil der vorliegenden Erfindung ist, dass die eingesetzten Hybridquats bereits bei geringen Einsatzmengen eine gute Wirkung entfalten.

Ein weiterer Vorteil ist, dass die eingesetzten Hybridquats in ökologischer Hinsicht wenig belastend sind.

Noch ein Vorteil ist es, dass die eingesetzten Hybridquats auf keratinischen Fasern eine leichtere Ausspülbarkeit zeigen als bisher bekannte quartäre Esterverbindungen.

Noch ein Vorteil der vorliegenden Erfindung ist es, dass die eingesetzten Hybridquats nicht auskristallisieren.

Noch ein Vorteil der vorliegenden Erfindung ist es, dass sie Haarfärbemittel vor dem Auswaschen schützt.

Noch ein Vorteil der vorliegenden Erfindung ist es, dass die Hybridquats sich leichter formulieren lassen.

Noch ein Vorteil der vorliegenden Erfindung ist es, dass sie die Hybridquats Kämmkräfte am nassen und am trockenen Haar reduzieren.

Ein weiterer Vorteil ist, dass sich die eingesetzten Hybridquats in hohen Aktivstoffgehalten einsetzen lassen.

Noch ein Vorteil der vorliegenden Erfindung ist es, dass die eingesetzten Hybridquats als Emulgatoren in O/W-Emulsionen ein hervorragendes Hautgefühl geben.

Noch ein Vorteil der vorliegenden Erfindung ist es, dass sie besonders wirtschaftlich ist.

Noch ein Vorteil der vorliegenden Erfindung ist es, dass die Formulierungen mit den Hybridquats antistatische Eigenschaften aufweisen.

### Gegenstand der vorliegenden Erfindung sind Zusammensetzungen enthaltend

### A) mindestens ein Hybridquat der allgemeinen Formel I)

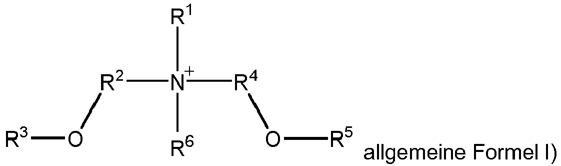

mit
R¹ ein Kohlenwasserstoffrest aufweisend 10 bis 32 Kohlenstoffatome, bevorzugt 12 bis 22 Kohlenstoffatome, insbesondere 16 bis 18 Kohlenstoffatome
R² und R⁴ unabhängig voneinander gleich oder verschieden ausgewählt aus der Gruppe der zweibindigen Kohlenwasserstoffreste aufweisend von 1 bis 4 Kohlenstoffatome, insbesondere C₂H₄ und C₃H₆,
R³ ein Acylrest einer Fettsäure mit einer Kettenlänge von 8 bis 32 Kohlenstoffatomen, bevorzugt 12 bis 22 Kohlenstoffatomen, insbesondere 16 bis 22 Kohlenstoffatomen, insbesondere bevorzugt 16 bis 18 Kohlenstoffatomen,
R⁵ H oder ein Acylrest einer Fettsäure mit einer Kettenlänge von 8 bis 32 Kohlenstoffatomen, bevorzugt 12 bis 22 Kohlenstoffatomen, insbesondere 16 bis 22 Kohlenstoffatomen, insbesondere bevorzugt 16 bis 18 Kohlenstoffatomen,
R⁶ ausgewählt aus der Gruppe der Kohlenwasserstoffreste aufweisend von 1 bis 4 Kohlenstoffatome, insbesondere Ethyl und Methyl,

### B) mindestens einen Fettalkohol.

Die erfindungsgemäßen Zusammensetzungen sind bevorzugt wässrige Zusammensetzungen.

Unter dem Begriff "wässrig" im Zusammenhang mit der vorliegenden Erfindung ist eine Zusammensetzung zu verstehen, die mindestens 5 Gew.-%, bevorzugt mindestens 30 Gew.-%, insbesondere mindestens 70 Gew.-%, Wasser, bezogen auf die betrachtete Gesamtzusammensetzung enthält.

Der "pH-Wert" im Zusammenhang mit der vorliegenden Erfindung ist definiert als der Wert, welcher für den entsprechenden Stoff bei 25 °C nach fünf Minuten Rühren mit einer gemäß ISO 4319 (1977) kalibrierten pH-Elektrode gemessen wird.

Alle angegebenen Prozent (%) sind, wenn nicht anders angegeben, Massenprozent.

Erfindungsgemäß bevorzugte Zusammensetzungen sind dadurch gekennzeichnet, dass
A) in einer Menge von 0,1 Gew.-% bis 10 Gew.-%, bevorzugt von 0,3 Gew.-% bis 5 Gew.-%, insbesondere von 0,5 Gew.-% bis 3 Gew.-%, und
B) in einer Menge von 1 Gew.-% bis 10 Gew.-%, bevorzugt von 2 Gew.-% bis 8 Gew.-%, insbesondere von 3 Gew.-% bis 6 Gew.-%,
enthalten ist,
wobei sich die Gewichtsprozente auf die Gesamtzusammensetzung beziehen.

### Es ist erfindungsgemäß bevorzugt, dass in dem mindestens einen Hybridquat der allgemeinen Formel I)

R¹ einen Kohlenwasserstoffrest von Fettalkohol darstellt.

Der Begriff "Kohlenwasserstoffrest von Fettalkohol" im Zusammenhang mit vorliegenden Erdfindung ist die überbleibende Struktur nach Streichung der OH-Gruppe des Fettalkohols. Bevorzugte Reste R¹ sind Kohlenwasserstoffreste von eine unverzweigten oder verzweigten Monoalkohol mit einer Alkylgruppe von 10 bis 30 C-Atomen, der auch ungesättigt sein kann. Bevorzugte Reste R¹ sind Kohlenwasserstoffreste von Decanol, Laurylalkohol, Isolaurylalkohol, Anteisolaurylalkohol, Myristylalkohol, Isomyristylalkohol, Cetylalkohol, Palmoleylalkohol, Stearylalkohol, Isostearylalkohol, Anteisostearylalkohol, Eicosanol, Petroselinylalkohol, Guerbetalkohol, Arachylalkohol, Gadoleylalkohol, Behenylalkohol, Erucylalkohol, Hectacosanol, Octacosanol, und Melissylalkohol, sowie von Mischungen davon, insbesondere von technischen Mischungen, vorzugsweise von technischen Kokos- oder Talgfettalkohole mit 12 bis 18, vorzugsweise mit 16 bis 18 Kohlenstoffatomen, sowie von den einfach ungesättigten Fettalkoholen, wie Oleylalkohol, Elaidylalkohol, Delta-9-cis-Hexadecenol, Delta-9-Octadecenol, trans-Delta-9-Octadecenol, cis-Delta-11-Octadecenol,trans-10,cis-12-Hexadecadien-1-ol, Octacosa-10,19-dien-1-ol und von mehrfach ungesättigten Fettalkoholen wie z.B. Linoleylalkohol (9Z, 12Z-Octadecadien-1-ol), Elaidolinoleylalkohol (9E, 12E-Octadecadien-1-ol), Linolenylalkohol (9Z, 12Z, 15Z-Octadecatrien-1-ol), Elaidolinolenylalkohol (9E, 12E, 15-E-Octadecatrien-1-ol), wobei Kohlenwasserstoffreste von Mischungen von Kokos- oder Talgfettalkohole mit 16 bis 18 Kohlenstoffatomen besonderes bevorzugt sind.

Bevorzugt ist R¹ der Kohlenwasserstoffrest eines Fettalkohols R¹-OH, der einen Schmelzpunkt von größer 25 °C, besonders bevorzugt größer 50 °C, bei 1 bar Druck aufweist.

### Es ist erfindungsgemäß bevorzugt, dass in dem mindestens einen Hybridquat der allgemeinen Formel I)

R¹ ein linearer Alkylrest aufweisend 16 bis 18 Kohlenstoffatome,
R² und R⁴ unabhängig voneinander gleich oder verschieden ausgewählt aus der Gruppe C₂H₄, und C3H6,
R³ ein Acylrest einer Fettsäure mit einer Kettenlänge von 16 bis 22, insbesondere 16 bis 18, Kohlenstoffatomen,
R⁵ H oder ein Acylrest einer Fettsäure mit einer Kettenlänge von 16 bis 22, insbesondere 16 bis 18, Kohlenstoffatomen,
R⁶ ausgewählt aus der Gruppe Ethyl und Methyl,
ist.

### Die erfindungsgemäßen Zusammensetzungen enthalten mindestens einen Fettalkohol (Komponente B).

Als Fettalkohol wird in diesem Zusammenhang bevorzugt ein unverzweigter oder verzweigter Monoalkohol mit einer Alkylgruppe von 8 bis 30 C-Atomen verstanden, der auch ungesättigt sein kann. Bevorzugte Fettalkohole sind Octanol, Decanol, Laurylalkohol, Isolaurylalkohol, Anteisolaurylalkohol, Myristylalkohol, Isomyristylalkohol, Cetylalkohol, Palmoleylalkohol, Stearylalkohol, Isostearylalkohol, Anteisostearylalkohol, Eicosanol, Petroselinylalkohol, Guerbetalkohol, Arachylalkohol, Gadoleylalkohol, Behenylalkohol, Erucylalkohol, Hectacosanol, Octacosanol, und Melissylalkohol,
sowie Mischungen davon, insbesondere technische Mischungen, vorzugsweise technische Kokos- oder Talgfettalkohole mit 12 bis 18, vorzugsweise mit 16 bis 18 Kohlenstoffatomen, sowie die einfach ungesättigten Fettalkohole, wie Oleylalkohol, Elaidylalkohol, Delta-9-cis-Hexadecenol, Delta-9-Octadecenol, trans-Delta-9-Octadecenol, cis-Delta-11-Octadecenol,trans-10,cis-12-Hexadecadien-1-ol, Octacosa-10,19-dien-1-ol und mehrfach ungesättigte Fettalkohole wie z.B. Linoleylalkohol (9Z, 12Z-Octadecadien-1-ol), Elaidolinoleylalkohol (9E, 12E-Octadecadien-1-ol), Linolenylalkohol (9Z, 12Z, 15Z-Octadecatrien-1-ol), Elaidolinolenylalkohol (9E, 12E, 15-E-Octadecatrien-1-ol), wobei Mischungen von Kokos- oder Talgfettalkohole mit 16 bis 18 Kohlenstoffatomen besonderes bevorzugt sind.

Bevorzugt ist ein Fettalkohol, der einen Schmelzpunkt von größer 25 °C, besonders bevorzugt größer 50 °C, bei 1 bar Druck aufweist.
Der Fettalkohol ist bevorzugt in einer Menge von 0,5 bis 20 Gew.-%, bevorzugt 1 bis 10 Gew.-%, insbesondere 2 bis 7 Gew.-%, in der erfindungsgemäßen Zusammensetzung enthalten, wobei sich die Gewichtsprozente auf die Gesamtzusammensetzung beziehen.

Es hat sich als vorteilhaft erwiesen, wenn die erfindungsgemäßen Zusammensetzungen zusätzlich eine Komponente C) Emulgator, insbesondere in einer Menge von 0,1 bis 10 Gew.-%, bevorzugt 0,25 bis 5 Gew.-%, insbesondere 0,5 bis 2,0 Gew.-%, enthalten, wobei sich die Gewichtsprozent auf die Gesamtzusammensetzung beziehen.
Bevorzugt enthaltene Emulgatoren sind nicht-ionische Emulgatoren.

In diesem Zusammenhang bevorzugte Emulgatoren sind ausgewählt aus der Gruppe der Fettalkoholalkoxylate, insbesondere der Fettalkoholethoxylate. Besonders bevorzugt enthaltene Fettalkoholethoxylate sind ausgewählt aus der Gruppe umfassend Polyoxyethylenether des Laurylalkohols, CAS-Nummer 9002-92-0, Macrogollaurylether, z. B. Polyoxyethylen (4) laurylether (Laureth-4, INCI),
Polyoxyethylen (9) laurylether Laureth-9 (INCI),
Polyoxyethylen (23) laurylether Laureth-23 (INCI)
Polyoxyethylenether des Cetylalkohols, CAS-Nummer 9004-95-9, z.B.
Polyoxyethylen (2) cetylether Ceteth-2 (INCI)
Polyoxyethylen (10) cetylether Ceteth-10 (INCI)
Polyoxyethylen (20) cetylether Ceteth-20 (INCI)
Polyoxyethylenether des Cetylstearylalkohols, CAS-Nummer 68439-49-6, z.B.
Polyoxyethylen (6) cetylstearylether Ceteareth-6 (INCI)
Polyoxyethylen (20) cetylstearylether Ceteareth-20 (INCI)
Polyoxyethylen (25) cetylstearylether Ceteareth-25 (INCI)
Polyoxyethylenether des Stearylalkohols, CAS-Nummer 9005-00-9, z.B.
Polyoxyethylen (2) stearylether Steareth-2 (INCI)
Polyoxyethylen (10) stearylether Steareth-10 (INCI)
Polyoxyethylen (20) stearylether Steareth-20 (INCI)
Polyoxyethylenether des Oleylalkohols, CAS-Nummer 9004-98-2, z.B.
Polyoxyethylen (2) oleylether Oleth-2 (INCI)
Polyoxyethylen (10) oleylether Oleth-10 (INCI)
Polyoxyethylen (20) oleylether Oleth-20 (INCI)
oder
Polyoxyethylen (10) tridecylether (CAS-Nummer 24938-91-8) und Trideceth-10 (INCI).

Alternativ bevorzugte Emulgatoren sind ausgewählt aus der Gruppe der Polyolester, insbesondere der Glycerinester und Polyglycerinester, insbesondere der Polyglycerinester. Bevorzugt enthaltene (Poly)glycerinester sind dadurch gekennzeichnet, dass sie Partialester sind. Besonders bevorzugte Polyglycerinpartialester sind ausgewählt aus der Gruppe umfassend

Polyglycerinpartialester wie in EP-B-0 835 862 beschrieben, die erhältlich sind durch Veresterung eines Polyglyceringemisches mit einem Veresterungsgrad des Polyglycerins zwischen 30 und 75 % und gesättigten oder ungesättigen, linearen oder verzweigten Fettsäuren mit 12 bis 22 C-Atomen und Dimerfettsäuren mit einer mittleren Funktionalität von 2 bis 2,4, Ester der Zitronensäure wie etwa der O/W-Emulgator Glycerylstearatcitrat, (2-Hydroxy-1,2,3-propantricarbonsäure-1,2,3-propantriolmonooctadecanoat, INCI Glyceryl Stearate Citrate, CAS 39175-72-9), der Citronensäureester des Glycerylstearats, u.a. unter der Bezeichnung AXOL C 62 im Handel erhältlich, Glycerylstearatcitrat wie in WO2006034992 und WO2008092676 beschrieben und Glyceryloleatcitrat wie in WO2004112731 beschrieben, ebenso einfache Polyglycerinester, wie beispielsweise Polygylcerin-3 Distearate, Polyglyceryl-10 Stearate, Polyglyceryl-6 Distearate, Mischester aus Polyglycerin und Methylglucose und Stearinsäure, wie beispielsweise Polyglyceryl-3 Methylglucose Distearate und (Poly-)glycerinpartialester mit einer oder mehrerer Carbonsäuren mit 10 bis 24 Kohlenstoffatomen und Resten einer polyfunktionalen Carbonsäure.

Prinzipiell können auch Sorbitan- oder Saccharoseester als Polyolester eingesetzt werden. Eine gängige Kombination ist beispielsweise Sorbitan Stearate & Sucrose Cocoate.

In einer weiteren Alternative bevorzugt enthaltene Emulgatoren sind ausgewählt aus der Gruppe der modifizierten Siloxane, beispielsweise solche, die neben aliphatischen Gruppen auf Basis von alpha-Olefinen auch Polyether tragen. Siloxanbasierte Emulgatoren für Öl-in-Wasser-Emulsionen müssen einen hydrophilen Charakter aufweisen, weshalb es sich in der Regel um reine Polyethersiloxane handelt. Besonders geeignete Beispiele sind relativ hydrophobe Polyethersiloxane wie in EP 1125574 beschrieben, hochmolekulare Polyethersiloxane wie in EP2168564 beschrieben und organomodifizierte Siloxanblockcopolymere wie in WO2009138306 beschrieben. Bevorzugt enthaltene modifizierte Siloxane sind dadurch gekennzeichnet, dass sie einen HLB-Wert >8 aufweisen. Besonders bevorzugte modifizierte Siloxane sind ausgewählt aus der Gruppe umfassend Bis-PEG/PPG-16/16 Dimethicone, PEG/PPG-16/16 Dimethicone, Bis-PEG/PPG-20/5 PEG/PPG-20/5 Dimethicone und Methoxy PEG/PPG-25/4 Dimethicone. Vorgenannte Emulgatoren ergeben im Zusammenhang mit der vorliegenden Erfindung besonders lagerstabile Formulierungen.

Bevorzugte erfindungsgemäße Zusammensetzungen sind Emulsionen, bevorzugt Öl in Wasser Emulsionen, besonders bevorzugt Emulsionen, bei denen die Ölphase bei 25 °C fest ist, somit liegt bei 25 °C eine Öl-in-Wasser Suspension vor.

Die erfindungsgemäßen Zusammensetzungen weisen vorteilhaft bei 25 °C einen pH-Wert von 1 bis 6,9, besonders bevorzugt von 2 bis 6,5 insbesondere von 2,5 bis 6 auf.

Es hat sich als vorteilhaft erwiesen, wenn die erfindungsgemäßen Zusammensetzungen zusätzlich eine Komponente D) Tensid, insbesondere in einer Menge von 0,1 bis 10 Gew.-%, bevorzugt 0,25 bis 5 Gew.-%, insbesondere 0,5 bis 2,0 Gew.-%, enthalten, wobei sich die Gewichtsprozent auf die Gesamtzusammensetzung beziehen.

Unter dem Begriff "Tensid" im Zusammenhang mit der vorliegenden Erfindung werden organische Substanzen mit grenzflächenaktiven Eigenschaften verstanden, die die Fähigkeit besitzen, die Oberflächenspannung von Wasser bei 20°C und bei einer Konzentration von 0,5 Gew.-% bezogen auf die Gesamtzusammensetzung auf unter 45 mN/m zu verringern. Die Oberflächenspannung wird hier durch die Ringmethode nach DuNoüy bei 25°C bestimmt.

Bei den Tensiden handelt es sich insbesondere um nicht-ionische Tenside, anionische Tenside, kationische Tenside und amphotere (zwitterionische) Tenside.

Es sind erfindungsgemäße Zusammensetzungen bevorzugt, die dadurch gekennzeichnet sind, dass das Tensid ausgewählt ist aus der Gruppe umfassend, bevorzugt bestehend aus: anionische Tenside, kationische Tenside und amphotere Tenside, wobei anionische Tenside und kationische Tenside besonders bevorzugt sind.

Enthält die erfindungsgemäße Zusammensetzung ein anionisches Tensid, sind insbesondere erfindungsgemäße Zusammensetzungen bevorzugt, die dadurch gekennzeichnet sind, dass das anionische Tensid ausgewählt ist aus der Gruppe umfassend, bevorzugt bestehend aus: Alkylsulfate in Form ihrer Alkali, Ammonium- oder Alkanolammoniumsalze, Alkylethersulfate, Alkylphosphate in Form ihrer Alkali, Ammonium- oder Alkanolammoniumsalze, Alkylethercarboxylate in Form ihrer Alkali- oder Ammoniumsalze,
Acylsarkosinate in Form ihrer Alkali- oder Ammoniumsalze,
Sulfosuccinate in Form ihrer Alkali- oder Ammoniumsalze und
Acylglutamate in Form ihrer Alkali- oder Ammoniumsalze,
wobei Alkylsulfate und Alkylethersulfate besonders bevorzugt sind.

Enthält die erfindungsgemäße Zusammensetzung ein kationisches Tensid, sind insbesondere erfindungsgemäße Zusammensetzungen bevorzugt, die dadurch gekennzeichnet sind, dass das kationische Tensid ausgewählt ist aus der Gruppe der quarternären Ammoniumverbindungen, bevorzugt bestehend aus Alkyltrimethylammoniumverbindungen, wobei Palmitamidopropyltrimonium Chloride besonders bevorzugt ist.

Enthält die erfindungsgemäße Zusammensetzung ein amphoteres Tensid, sind insbesondere erfindungsgemäße Zusammensetzungen bevorzugt, die dadurch gekennzeichnet sind, dass das amphotere Tensid ausgewählt ist aus der Gruppe umfassend, bevorzugt bestehend aus: Betaine, Amphoacetate und Amphopropionate, N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat, Verbindungen, die außer einer C8/18-Alkyl- oder -Acylgruppe im Molekül mindestens eine freie Aminogruppe und mindestens eine -COOH- oder -SO₃H- Gruppe enthalten und zur Ausbildung innerer Salze befähigt sind, wie zum Beispiel N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe, N-Kokosalkylaminopropionat, Kokosacylaminoethylaminopropionat und C12/18-Acylsarcosin, wobei N-Acylaminopropyl-N,N-dimethylammoniumglycinate besonders bevorzugt sind.

Die erfindungsgemäßen Zusammensetzungen können z.B. mindestens eine weitere, zusätzliche Komponente enthalten, ausgewählt aus der Gruppe der
Emollients,
Co-Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie der eingesetzten Mengen dieser Komponenten wird ausdrücklich auf die dem Fachmann bekannten einschlägigen Handbücher, z. B. K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Die in den erfindungsgemäßen Zusammensetzungen enthaltenen Hybridquats der allgemeinen Formel I) sowie die erfindungsgemäßen Zusammensetzungen lassen sich erfindungsgemäß für die kosmetische Behandlung von Haut oder keratinischer Fasern, insbesondere für die Haarbehandlung, besonders bevorzugt zur Konditionierung von Haaren, verwenden.

In diesem Zusammenhang werden bevorzugt solche Hybridquats der allgemeinen Formel I) bevorzugt verwendet, die oben als bevorzugt in den erfindungsgemäßen Zusammensetzungen enthalten beschrieben werden.

Die erfindungsgemäße Verwendung führt zur Verbesserung der Konditionierung, des Glanzes, der Flexibilität, der Spannkraft, und/oder der Kämmbarkeit sowie einer Reduzierung der Bruchwahrscheinlichkeit der behandelten Faser und außerdem reduziert sie die statischen Kräfte zwischen den Fasern.

Noch ein Gegenstand der vorliegenden Erfindung ist die Verwendung der in den erfindungsgemäßen Zusammensetzungen enthaltenen Hybridquats der allgemeinen Formel I) zur Viskositätsmodifizierung, insbesondere Verdickung, einer mindestens einen Fettalkohol enthaltenen Zusammensetzung.

In diesem Zusammenhang werden bevorzugt solche Hybridquats der allgemeinen Formel I) bevorzugt verwendet, die oben als bevorzugt in den erfindungsgemäßen Zusammensetzungen enthalten beschrieben werden.

Noch ein Gegenstand der vorliegenden Erfindung ist die Verwendung von den in den erfindungsgemäßen Zusammensetzungen enthaltenen Hybridquats als Emulgator, insbesondere kationischem Emulgator, in bevorzugt kosmetischen Formulierungen, insbesondere unter Erhalt von O/W-Emulsionen.

In diesem Zusammenhang werden bevorzugt solche Hybridquats der allgemeinen Formel I) bevorzugt verwendet, die oben als bevorzugt in den erfindungsgemäßen Zusammensetzungen enthalten beschrieben werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:

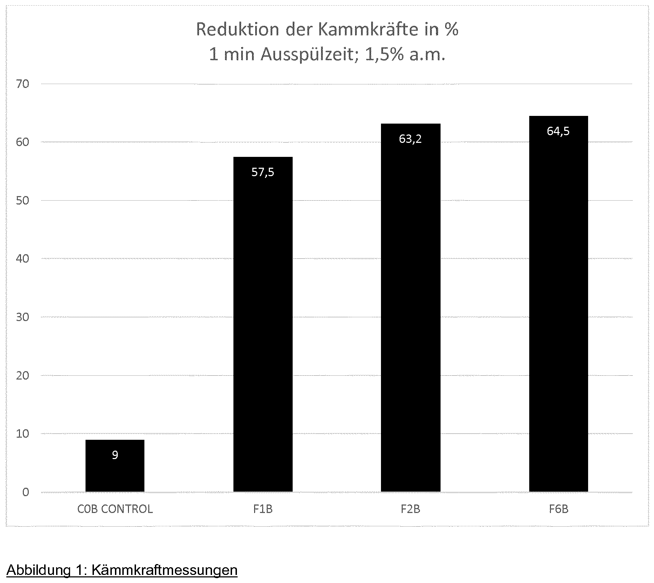

Abbildung 1: Kämmkraftmessungen

### Beispiele:

**Tabelle A: Amine mit zwei Hydroxyethyl-Gruppen und Fettalkoholresten R¹**

| Amin | R¹ (alle % Angaben sind mol %) | R² | R⁴ | Handelsname | Quelle |
|---|---|---|---|---|---|
| A1 | Stearyl mit 93 % C18, 5 % C16, 2 % > C20 mit IZ =0-3,0 | C₂H₄ | C₂H₄ | Varonic S 202 | Evonik Corporation, USA |
| A2 | Talg mit 3 % C14, 30 % C16, 67 % C18 mit IZ = 38,0-54,0 | C₂H₄ | C₂H₄ | Varonic T 202 | Evonik Corporation, USA |
| A3 | Hydr. Talg mit 3 % C14, 30 % C16, 67 % C18 mit IZ <= 3,0 | C₂H₄ | C₂H₄ | Varonic U 202 | Evonik Corporation, USA |

**Tabelle B: Reste R³ bzw. gegebenenfalls R⁵ bestimmende Fettsäure**

| Fettsäure | Quelle | Iodzahl |
|---|---|---|
| FS1 | Kokosfettsäure, Wilfarin DC-0818, Wilmar | < 12 |
| FS2 | Stearinfettsäure, pflanzlich, Pristeren 4928, Croda | < 1,0 |
| FS3 | Pflanzenmischölfettsäure, Nouracid IF 10, Oleon | 110 - 125 |
| FS 4 | 12-Hydroxystearinsäure, Alberdingk Boley | < 5 |

### Beispiel B1 bis B6:

482,8 g (1,325 mol) Amin A1 wurden in einen Dreihalskolben mit Kolonne, Destillationsbrücke und Rührmotor gegeben und unter Stickstoffatmosphäre auf 80 °C aufgeheizt. Dazu wurden 332,8 g (1,59 mol) Fettsäure FS 1 gegeben sowie 0,4 g 50%ige wässerige Hypophosphorige Säure. Es wurde ein Vakuum von 100 mbar angelegt und vorsichtig auf 195 °C erwärmt, wobei Reaktionswasser im Abgang der Destillationsbrücke aufgefangen wurde. Nach 3 Stunden wurde das Vakuum auf 20 mbar verringert und über 2 Stunden weiterreagiert. Das so erhaltene Kondensationsprodukt hatte eine Säurezahl von 1,3 mg KOH/g und eine Aminzahl von 94,0 mg KOH/g. Die Reaktionsmischung wurde auf 80 °C abgekühlt. Unter Rühren wurden innerhalb einer Stunde 155,4 g (1,232 mol) Dimethylsulfat zu getropft, wobei die Temperatur in einem Bereich von 80 -95 °C gehalten wurde. Anschließend wurden 103 g wasserfreies Ethanol zugegeben und eine Stunde bei 80 °C nachgerührt. Das so erhaltene Produkt wies eine Aminzahl von 3,0 mg KOH/g auf.

Die weiteren Beispiele B2-B6 und Vergleichsbeispiele wurden entsprechend dieser Vorschrift jedoch mit variierten Edukten bzw. variierten Mengenverhältnissen - jeweils wie in der nachfolgenden Tabelle C angegeben - durchgeführt.

**Tabelle C:**

| Beispiel | Amin | Fettsäure | Jodzahl | Mol Fettsäure pro Mol Amin | Versuchsnummer |
|---|---|---|---|---|---|
| B1 | A1 | FS1 | < 12 | 1.2 | TS 324/16 |
| B2 | A1 | FS2 | < 1.0 | 0.8 | AE 323//16 |
| B3 | A1 | FS3 | 110 -125 | 0.8 | MK 320/16 |
| B4 | A1 | FS3 | 110-125 | 1.5 | TS 321/16 |
| B5 | A3 | FS2 | < 1.0 | 1.5 | TS 13/17 |
| B6 | A2 | FS4 | < 5 | 1.0 | TS 252/16 |

### Beispiel 2: Anwendungstechnik von Haarbehandlungsmitteln unter Verwendung von Beispielen B1, B2 und B6 sowie kommerziellen Marktprodukten.

Für die anwendungstechnische Beurteilung wurden Haartressen verwendet, die durch eine Bleichbehandlung standardisiert vorgeschädigt wurden. Dazu werden friseurübliche Produkte eingesetzt. Die Schädigung der Haartressen wird im Detail in der DE10327871 beschrieben. Für die anwendungstechnische Beurteilung wurde die erfindungsgemäße Verbindungen aus Beispiel B1, B2 und B6 - in einer einfachen kosmetischen Formulierung eingesetzt.

Als Referenzverbindungen wurden das kommerziell erhältliche Alkylquat (INCI) Behentrimonium Chloride (VARISOFT® BT 85 Pellets, Evonik Nutrition & Care) und das kommerziell erhältliche Esterquat (INCI) Distearoylethyl Hydroxyethylmonium Methosulfate (VARISOFT® EQ F 75, Evonik Nutrition & Care) verwendet.

Die Anwendungseigenschaften beim Einsatz in Haarspülungen wurden in den folgenden Rezepturen überprüft (Tab. 1):

**Tab. 1: Haarspülung-Formulierungen zur Austestung der haarkonditionierenden Eigenschaften**

| Formulierungsbeispiele | C0a | F1a | F2a | F5a | F6a | V7a | V8a |
|---|---|---|---|---|---|---|---|
| TEGINACID® C, Evonik Industries (INCI: Ceteareth-25) | 0,5% | 0,5% | 0,5% | 0,5% | 0,5% | 0,5% | 0,5% |
| TEGO®Alkanol 1618, Evonik Nutrition & Care (INCI: Cetearylalkohol) | 5% | 5% | 5% | 5% | 5% | 4,67% | 5% |
| Beispiel B1 (90%) | | 1,1% | | | | | |
| Beispiel B2 (90%) | | | 1,1% | | | | |
| Beispiel B5 (90%) | | | | 1,1% | | | |
| Beispiel B6 (90%) | | | | | 1,1% | | |
| VARISOFT® EQ F 75, (75% in Cetearylalkohol), Evonik Nutrition & Care (INCI: Distearoylethyl Hydroxyethylmonium Methosulfate) | | | | | | 1,33% | |
| VARISOFT® BT 85, (85%ig in Isopropanol), Evonik Nutrition & Care (INCI: Behentrimonium Chloride) | | | | | | | 1,17% |
| Wasser, demineralisiert | | | | | | | |
| Zitronensäure | | | | | | | |

Die Zusammensetzung der Testformulierungen ist zur Vermeidung der Beeinflussung der Testergebnisse durch (normalerweise vorhandene) Formulierungsbestandteile bewusst einfach gewählt. Erfindungsgemäße Formulierungen können neben den genannten Inhaltsstoffen und/oder anstatt der genannten Inhaltsstoffe noch weitere Inhaltsstoffe enthalten. Insbesondere die Kombination mit weiteren Inhaltsstoffen kann bei den beschriebenen Effekten zu einer synergistischen Verbesserung führen.

Die Vorbehandlung der Haare erfolgt durch eine Shampooformulierung (Tab. 2), welche keine Konditioniermittel enthält.

**Tab. 2: Shampooformulierung für die Vorbehandlung der Haartressen.**

| | |
|---|---|
| Texapon NSO®, 28%-ig, Cognis (INCI: SodiumLaureth Sulfate) | 42,9% |
| NaCl | 3% |
| Wasser, demineralisiert | ad 100,0 |

Standardisierte Behandlung von vorgeschädigten Haarsträhnen mit konditionierenden Proben: Die wie oben beschrieben vorgeschädigten Haarsträhnen werden mit der Shampooformulierung aus Tab. 2 gewaschen.

Hierbei werden die Haarsträhnen unter fließendem, warmem Wasser benetzt. Das überschüssige Wasser wird leicht von Hand ausgedrückt, dann wird das Shampoo aufgebracht und 1 min lang sanft ins Haar eingearbeitet (0.5 ml/2 g Haarsträhne). Die Haartresse wird für 30 s unter fließendwarmem Wasser ausgespült. Diese Prozedur wird ein weiteres Mal wiederholt, nur dass abschließend 1 min lang ausgespült wird.

Anschließend werden direkt nach dem Waschen die Haarsträhnen mit den Haarspülungs-Formulierungen aus Tab. 1 konditioniert.

Hierbei wird die Spülung aufgebracht und sanft ins Haar eingearbeitet (0.5 ml/2 g Haarsträhne). Nach einer Verweilzeit von 1 min wird das Haar für a) 1 min oder für b) 3 min gespült.

Vor der Beurteilung der Trockensensorik wird das Haar an der Luft bei 50% Luftfeuchtigkeit und 25°C für mindestens 12 h getrocknet.

### Beurteilungskriterien:

Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.

### Die Testkriterien sind:

Nasskämmbarkeit, Nassgriff, Trockenkämmbarkeit, Trockengriff.

In den folgenden Tabellen werden die Ergebnisse der sensorischen Beurteilung der wie oben beschrieben durchgeführten Behandlung der Haarsträhnen bei a) 1 min Ausspülzeit und bei b) 3 min Ausspülzeit mit den erfindungsgemäßen Formulierungen F1a, F2a, F6a, den Vergleichsformulierungen V7a, V8a und der Kontrollformulierung C0a (Control ohne Testsubstanz) verglichen

### a) 1 min Ausspülzeit

**Tab. 3a: Ergebnisse der Konditionierung von Haar bei 1 min Ausspülzeit**

| | Nasskämmbarkeit | Nassgriff | Trockenkämmbarkeit | Trockengriff |
|---|---|---|---|---|
| Erfindungsgemäße Formulierung F1a | 4,5 | 4,5 | 5 | 5 |
| Erfindungsgemäße Formulierung F2a | 4 | 4 | 5 | 5 |
| Erfindungsgemäße Formulierung F3a | 4 | 4 | 5 | 5 |
| Vergleichsformulierung V7a (F 75) | 3,5 | 3,5 | 3,5 | 3 |
| Vergleichsformulierung V8a (BT 85) | 4 | 4 | 4 | 4 |
| Kontrolle C0a | 2 | 2 | 3 | 2,5 |

### b) 3 min Ausspülzeit

**Tab. 3b: Ergebnisse der Konditionierung von Haar bei 3 min Ausspülzeit**

| | Nasskämmbarkeit | Nassgriff | Trockenkämmbarkeit | Trockengriff |
|---|---|---|---|---|
| Erfindungsgemäße Formulierung F1a | 4 | 4 | 5 | 5 |
| Erfindungsgemäße Formulierung F2a | 4 | 4 | 4,5 | 5 |
| Erfindungsgemäße Formulierung F3a | 4 | 4 | 5 | 5 |
| Vergleichsformulierung V7a (F 75) | 3 | 3 | 3 | 3 |
| Vergleichsformulierung V8a (BT 85) | 4 | 4 | 4 | 4 |
| Kontrolle C0a | 1,5 | 2 | 2,5 | 2 |

Die Ergebnisse in Tabelle 3a zeigen, dass die erfindungsgemäßen Formulierungen F1a, F2a, und F6a jeweils im Vergleich mit den Formulierungen V7a, und V8a sowohl bezüglich der Kämmbarkeiten und Griffes bei nassem und trockenem Haar besser abschneiden. Insbesondere bei trockenem Haar sind die Absolutwerte der Hybridquats ausgezeichnet. Überraschenderweise zeigen die Hybridquats bei 3 Minuten Ausspülzeit kaum eine Verschlechterung der Ergebnisse. Insbesondere auf dem trockenen Haar bleibt die Konditionierleistung sehr hoch. Alle erfindungsgemäßen Formulierungen sind gegenüber den Vergleichsformulierungen als signifikant besser einzustufen.

### Beispiel 3: Einfluss der erfindungsgemäßen Verbindungen auf Kämmkräfte von Haaren

### Versuchsbedingungen:

Messgerät: Diastron MTT 175
Messstrecke: 20 cm
Kämmgeschwindigkeit: 2000 mm/min
Verwendete Haarsträhnen: Länge = 23 cm; Breite = 1,5cm; Gewicht = 2 g Messbedingungen: T = 22°C.

Die Haarsträhnen werden mit einer Restfeuchte von 60%, bestimmt durch Gewichtsbestimmung, gemessen.

Für die Versuche wird europäisches, ungeschädigtes, dunkelbraunes Haar eingesetzt. Zur Durchführung der Kämmkraftmessungen wird dieses Haar im Labor nach Standardbedingungen durch Dauerwelle geschädigt:
1.) 4 g Dauerwell-Lösung/g Haar, 15 min. einwirken lassen, 2 min. ausspülen unter fließendem Leitungswasser (T = 35°C). (Dauerwell-Lösung: Universal-Dauerwelle, Basler) 2.) 4 g Fixierer (1 Teil Fixierlösung. + 3 Teile Wasser)/g Haar, 10 min. einwirken lassen, 2 min. ausspülen. (Fixierlösung: Schaumfixierung Konzentrat, Basler)
   Durchführung der Kämmkraftmessung vor der Behandlung mit der Testformulierung:
   Die vorgeschädigten Haarsträhnen werden über Nacht klimatisiert.
3.) Die Haarsträhne wird 1 min in einer Pufferlösung (Na-Citrat, pH = 6) eingetaucht. 4.) Die Haarsträhne wird so lange von Hand vorgekämmt, bis keine Änderung des Kämmwiderstandes festzustellen ist. 5.) Die Haarsträhne wird am Messgerät befestigt und die erste Kämmkraftmessung wird durchgeführt. Die Messung wird insgesamt 10 mal wiederholt.

### Behandlung der Strähnen:

Pro Haarsträhne werden 0,5 g der jeweiligen Testformulierung verwendet (2 g Haar/0,5 g Lösung). Die Formulierung wird 30 sec. in das Haar einmassiert und dann für 5 min. liegen gelassen, anschließend 1 min. bzw. 3 min. unter fließendem Leitungswasser abgespült.

Zur Durchführung der Kämmkraftmessung nach der Behandlung mit der Testformulierung werden die Punkte 3-5 wiederholt.

Anschließend wird die Kämmarbeit (%) vor und nach der Behandlung mit der Testformulierung berechnet.

### Verwendete Testformulierungen:

Die Kämmkräfte beim Einsatz in Haarspülungen wurden in den folgenden Rezepturen überprüft (Tab. 4):

**Tab. 4: Haarspülung-Formulierungen zur Austestung der Kämmkräfte und der Antistatik**

| Formulierungsbeispiele | C0b | F1b | F2b | F6b | V7b | V8b |
|---|---|---|---|---|---|---|
| TEGINACID® C, Evonik Industries (INCI: Ceteareth-25) | 0,5% | 0,5% | 0,5% | 0,5% | 0,5% | 0,5% |
| TEGO®Alkanol 1618, Evonik Nutrition & Care (INCI: Cetearylalkohol) | 5% | 5% | 5% | 5% | 4,5% | 5% |
| Beispiel B1 (90%) | | 1,65% | | | | |
| Beispiel B2 (90%) | | | 1,65% | | | |
| Beispiel B6 (90%) | | | | 1,65% | | |
| VARISOFT® EQ F 75, (75% in Cetearylalkohol), Evonik Nutrition & Care (INCI: Distearoylethyl Hydroxyethylmonium Methosulfate) | | | | | 2% | |
| VARISOFT® BT 85, (85%ig in Isopropanol), Evonik Nutrition & Care (INCI: Behentrimonium Chloride) | | | | | | 1,75% |
| Wasser, demineralisiert | | | | | | |
| Zitronensäure | | | | | | |

In der Abbildung 1 werden die Ergebnisse der Kämmkraftmessungen der wie oben beschrieben durchgeführten Experimente bei 1 min Ausspülzeit mit den erfindungsgemäßen Formulierungen F1b, F2b, F6b, sowie der Kontrollformulierung C0b (Kontrolle ohne Testsubstanz) verglichen. Die Ergebnisse in Abbildung 1 zeigen, dass die erfindungsgemäßen Formulierungen F1b, F2b, F6b bei 1 min Ausspülzeit eine starke Reduktion der Kämmkräfte aufweisen.

### Beispiel 4: Antistatische Ausrüstung von keratinischen Fasern

Zur Austestung des antistatischen Verhaltens wurde die Schattensilhouette-Methode angewendet. Die oben beschriebenen, vorbehandelten Haartressen, ein Plastikkamm, ein Punktstrahler und ein mit konzentrischen Halbkreisen gekennzeichnetes Projektionsfeld werden eingesetzt.

Die Versuche wurden unter standardisierten klimatischen Bedingungen durchgeführt. Die Haartresse wird im Abstand von 15 cm vom Projektionsfeld aufgehängt. Der Punktstrahler wird in einer Entfernung von 145 cm von der Haartresse aufgestellt, so dass ein Schatten auf das Projektionsfeld fällt.

Nun wird die Haartresse mit dem Kamm fünfmal hintereinander gekämmt. Die elektrostatische Aufladung wird über die Schattensilhouette gemessen, indem die beiden äußeren Punkte des Schattens markiert werden und deren Abstand ermittelt wird. Je kleiner die Schattenfläche, umso effektiver ist die antistatische Wirkung.

### Ergebnis:

| Formulierung (s. Tabelle 4) | Abstand |
|---|---|
| C0b | 15 cm |
| F1b | 7 cm |
| F2b | 7,5 cm |
| F6b | 6,5 cm |
| V7b | 9 cm |
| V8b | 7,5 cm |

### Beispiel 5: Viskositäten der Beispielformulierungen

### Ergebnis:

| Formulierung (s. Tabelle 1) | Brookfield Viskosität 25°C (Sp. T-C, 30 rpm) |
|---|---|
| C0b | 5550 |
| F1a | 15000 |
| F2a | 30000 |
| F5a | 32000 |
| F6a | 23600 |
| V7a | 11000 |
| V8a | 12000 |

Die erfindungsgemäßen Formulierungen F1a, F2a, F5a and F6a zeigen signifikant höhere Viskositäten im Vergleich zu den entsprechenden Formulierungen auf Basis der als sehr gute Emulgatoren bekannten VARISOFT® EQ F 75 und VARISOFT® BT 85.

## Patentansprüche

1. Zusammensetzung enthaltend
A) mindestens ein Hybridquat der allgemeinen Formel I) 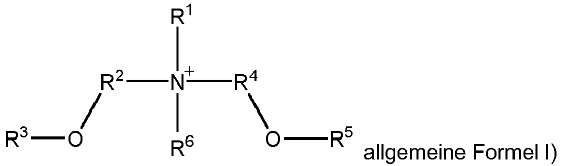 mit
R¹ ein Kohlenwasserstoffrest aufweisend 10 bis 32 Kohlenstoffatome, bevorzugt 12 bis 22 Kohlenstoffatome, insbesondere 16 bis 18 Kohlenstoffatome
R² und R⁴ unabhängig voneinander gleich oder verschieden ausgewählt aus der Gruppe der zweibindigen Kohlenwasserstoffreste aufweisend von 1 bis 4 Kohlenstoffatome, insbesondere C₂H₄ und C₃H₆,
R³ ein Acylrest einer Fettsäure mit einer Kettenlänge von 8 bis 32 Kohlenstoffatomen, bevorzugt 12 bis 22 Kohlenstoffatomen, insbesondere 16 bis 22 Kohlenstoffatomen, insbesondere bevorzugt 16 bis 18 Kohlenstoffatomen,
R⁵ H oder ein Acylrest einer Fettsäure mit einer Kettenlänge von 8 bis 32 Kohlenstoffatomen, bevorzugt 12 bis 22 Kohlenstoffatomen, insbesondere 16 bis 22 Kohlenstoffatomen, insbesondere bevorzugt 16 bis 18 Kohlenstoffatomen,
R⁶ ausgewählt aus der Gruppe der Kohlenwasserstoffreste aufweisend von 1 bis 4 Kohlenstoffatome, insbesondere Ethyl und Methyl,
B) mindestens einen Fettalkohol.

2. Zusammensetzung nach Anspruch 2, **dadurch gekennzeichnet, dass**
A) in einer Menge von 0,1 Gew.-% bis 10 Gew.-%, bevorzugt von 0,3 Gew.-% bis 5 Gew.-%, insbesondere von 0,5 Gew.-% bis 3 Gew.-%, und
B) in einer Menge von 1 Gew.-% bis 10 Gew.-%, bevorzugt von 2 Gew.-% bis 8 Gew.-%, insbesondere von 3 Gew.-% bis 6 Gew.-%,
enthalten ist,
wobei sich die Gewichtsprozente auf die Gesamtzusammensetzung beziehen.

3. Zusammensetzung nach Anspruch 1 oder 2, **dadurch gekennzeichnet, dass** in dem mindestens einen Hybridquat der allgemeinen Formel I) der Rest R¹ einen Kohlenwasserstoffrest von Fettalkohol darstellt.

4. Zusammensetzung nach mindestens einem der vorherigen Ansprüche, **dadurch gekennzeichnet, dass** in dem mindestens einen Hybridquat der allgemeinen Formel I)
R¹ ein linearer Alkylrest aufweisend 16 bis 18 Kohlenstoffatome,
R² und R⁴ unabhängig voneinander gleich oder verschieden ausgewählt aus der Gruppe C₂H₄ und C₃H₆,
R³ ein Acylrest einer Fettsäure mit einer Kettenlänge von 16 bis 22, insbesondere 16 bis 18, Kohlenstoffatomen,
R⁵ H oder ein Acylrest einer Fettsäure mit einer Kettenlänge von 16 bis 22, insbesondere 16 bis 18, Kohlenstoffatomen,
R⁶ ausgewählt aus der Gruppe Ethyl und Methyl,
ist.

5. Zusammensetzung nach mindestens einem der vorherigen Ansprüche, **dadurch gekennzeichnet, dass** B) der mindestens eine Fettalkohol ausgewählt ist aus den Fettalkoholen Octanol, Decanol, Laurylalkohol, Isolaurylalkohol, Anteisolaurylalkohol, Myristylalkohol, Isomyristylalkohol, Cetylalkohol, Palmoleylalkohol, Stearylalkohol, Isostearylalkohol, Anteisostearylalkohol, Eicosanol, Petroselinylalkohol, Guerbetalkohol, Arachylalkohol, Gadoleylalkohol, Behenylalkohol, Erucylalkohol, Hectacosanol, Octacosanol, und Melissylalkohol.

6. Verwendung mindestens eines Hybridquats der allgemeinen Formel I) wie in mindestens einem der Ansprüche 1 bis 5 beschrieben oder einer Zusammensetzung nach mindestens einem der Ansprüche 1 bis 5 zur Konditionierung von Haaren.

7. Verwendung mindestens eines Hybridquats der allgemeinen Formel I) wie in mindestens einem der Ansprüche 1 bis 5 beschrieben zur Viskositätsmodifizierung einer mindestens einen Fettalkohol enthaltenen Zusammensetzung.
